# EUROPEAN PATENT APPLICATION

(11) **EP 3 327 597 A1**
(43) Date of publication of application: **30.05.2018**
(21) Application number: 16200410.5
(22) Date of filing: 24.11.2016
(51) Int. Cl.: G06F 19/00, A63B 21/00

(54) **PHYSICAL EXERCISE APPARATUS AND METHOD FOR MOTIVATING A USER TO INTERACT WITH AN EXERCISE FACILITY**

(71) Applicant: Kucher, Anna, Manchester M4 1PY (GB)
(72) Inventor: Kucher, Anna, Manchester M4 1PY (GB)
(74) Representative: Stolmár & Partner Patentanwälte PartG mbB

(57) **Abstract**

A physical exercise apparatus (1; 1'; 1 "; 1'") for motivating a user (2), comprising: an exercise facility (10), a measuring device (20) adapted to determine a variable during the physical exercise of the user (2), a processing device (30) coupled to the measuring device (20) and adapted to produce moving graphics with a content depending on the determined variable, and a display device (40) coupled to the processing device (30) and adapted to display the produced moving graphics, wherein: producing the moving graphics comprises adjusting the size of an object in the content of the moving graphics to give the user (2) the impression that the object changes its position relative to the user's position, and the displayed moving graphics increases the interaction of the user (2) with the exercise facility. A physical exercising method for motivating a user to interact with an exercise facility.

## Description

### Field of the invention

The present invention generally relates to the field of physical exercise apparatuses and methods, in particular to apparatuses and methods which are adapted particularly to motivate a user to exercise.

### Background of the invention

Following increasing urbanization in all parts of the world, more and more people are forced to exercise indoors. It is, therefore, no surprise that professional gyms face a constant influx of athletes of various training levels. Likewise, the market for fitness devices that can be used at home, i.e., home exercise devices, has increased substantially over the past decades.

While modern gyms offer a variety of facilities for different kinds of physical exercise, the regular user will find exercising there for an extended period of time slightly tedious, due to the repetitiveness of the movements and never changing surroundings. The same holds true for home exercise devices. Modern technology, such as TV screens directly built into the exercise devices, enables users to consume their favourite TV show while, for example, cycling on a stationary exercise bike. This certainly makes the exercising experience more pleasant for the user. However, such distraction does not lead to an increased exercise intensity. To the contrary, in most cases it will actually decrease the interaction of the user with the exercise device.

WO 2015/132683 discloses an exercise arrangement and methods, wherein the exercise routine is conducted in an exercise zone and the user is presented with a transmission with visual or audible prompts to perform movements. This method and the exercise arrangement are, however, not readily adjustable.

Therefore, there is a long felt need for further exercise devices providing a motivation to the user that is inherently linked to the exercise itself.

### Summary of the invention

The invention solves this problem by providing a physical exercise apparatus and method as described by the independent claims.

In particular, in an aspect of the invention, the physical exercise apparatus for motivating a user comprises an exercise facility, a measuring device adapted to determine a variable during the physical exercise of the user, a processing device coupled to the measuring device and adapted to produce moving graphics with a content depending on the determined variable, and a display device coupled to the processing device and adapted to display the produced moving graphics, wherein producing the moving graphics comprises adjusting the size of an object in the content of the moving graphics to give the user the impression that the object changes its position relative to the user's position, and the displayed moving graphics increases the interaction of the user with the exercise facility.

In another aspect, the object is representative of a pursuer pursuing the user. Thus, fear is induced in the user which makes the user run exercise harder involuntarily.

In another aspect, the size of the object is inversely proportional to the determined variable. Preferably, the size of the object depends on a difference between the determined variable and a pre-set value. Accordingly, the physical exercise apparatus according to the present invention provides an easily understandable representation of various variables related to the physical exercise.

In another aspect of the invention, the variable may be representative of a heart rate, velocity, sweat factor and / or temperature of the user. Alternatively, the variable may be representative of a duration and / or intensity of the physical exercise. Since many different variables can be measured, the physical exercise apparatus according to the invention is very flexible and easy to adjust.

The displayed moving graphics may be adapted to increase an adrenaline level in the user. This may further enhance the effect of the physical effect on the user's body.

In another aspect, the processing device is further adapted to produce sound dependent on the determined variable, and a sound transmitting device coupled to the processing device and adapted to transmit sound to the user, thereby creating an immersive environment for the physical exercise.

In another aspect, the processing device is further adapted to produce a haptic feedback dependent on the determined variable, and a haptic feedback device coupled to the processing device and adapted to give a haptic feedback to the use, thereby further promoting the feeling of the user to be living an adventure.

Preferably, the physical exercise apparatus according to the invention may further comprise an input device coupled to the processing device and adapted to receive a scenario selected by the user from a plurality of scenarios presented to the user, so that the user's motivation is further increased since he / she is able to create a scenario that interests him / her.

Preferably, the display device of the physical exercise apparatus may be located alongside, above, underneath or behind the user. Different locations of the display device 40 can enhance the visibility of the displayed graphics for the user.

In yet another aspect, a physical exercise method for motivating a user to interact with an exercise facility comprises a) determining, with a measuring device, a variable during a physical exercise of the user, b) producing, with a processing device, moving graphics with a content depending on the determined variable, and c) displaying the adapted moving graphics to the user, wherein producing the moving graphics involves adjusting the size of an object forming the content of the moving graphics to give the user the impression that the object changes its position relative to the user's position, and wherein the displayed moving graphics increases the interaction of the user with the exercise facility.

In a preferred aspect, the object forming the content of the moving graphics is representative of a pursuer pursuing the user. The fear induced by the pursuer further motivates the user to give his / her best during the physical exercise.

The size of the object may be inversely proportional to the determined variable. The size of the object may also depend on a difference between the determined variable and a pre-set value. This allows the user to visualize the abstract values related to the physical exercise.

In another aspect of the invention, the variable may be representative of a heart rate, velocity, sweat factor and / or temperature of the user. Alternatively, the variable may be representative of a duration and / or intensity of the physical exercise. Thus, the method of the present invention is very flexible and can be adjusted to various needs.

### Brief description of the drawing

Further characteristics and advantages of the present invention will be more evident from a review of the following specification of preferred, but not exclusive, embodiments, shown for illustration purposes only and without limitation, with the aid of the attached drawing, in which:
Fig. 1 shows an exemplary set-up of a physical exercise apparatus 1 according to an embodiment of the present invention;

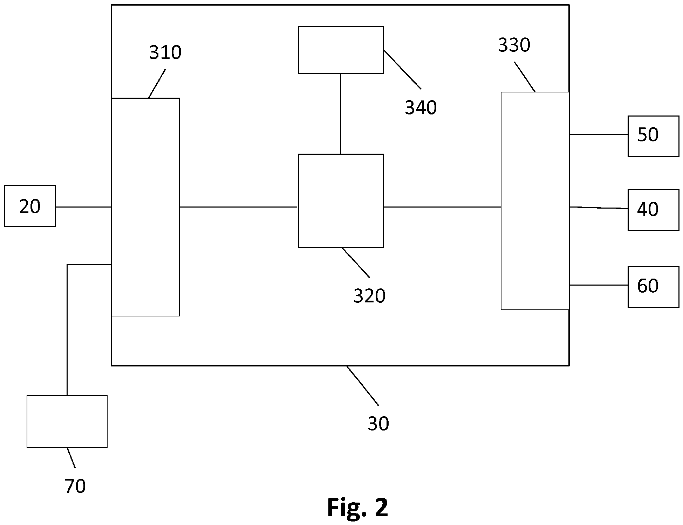

Fig. 2 shows the processing device 30 according to an embodiment of the present invention;
Fig. 3 shows an embodiment of the present invention comprising a treadmill 10' and a screen 40';
Fig. 4 shows another embodiment of the present invention comprising a swimming pool 10" and a projector 40"; and
Fig. 5 shows yet another embodiment of the present invention comprising a climbing wall 10'" and a movable screen 40"'.

### Detailed description of the invention

The term "coupled" as used herein is understood to include both physical coupling, i.e., via a cable, and virtual coupling, e.g., via a wireless connection, radio connection, Bluetooth connection, etc.

In a first aspect, the present invention provides a physical exercise apparatus 1 for motivating a user 2, comprising an exercise facility 10, a measuring device 20 adapted to determine a variable during the physical exercise of the user 2, a processing device 30 coupled to the measuring device 20 and adapted to produce moving graphics with a content depending on the determined variable, and a display device 40 coupled to the processing device 30 and adapted to display the produced moving graphics, wherein producing the moving graphics comprises adjusting the size of an object in the content of the moving graphics to give the user 2 the impression that the object changes its position relative to the user's position, and the displayed moving graphics increases the interaction of the user 2 with the exercise facility 10.

The physical exercise apparatus 1 according to the invention, thus, provides a translation of the abstract variables of the physical exercise into values that can be represented visually and easily understood. Since the user has a better feeling for his / her achievement during the physical exercise, he / she is consequently more motivated to exercise.

"Physical exercise" as used herein is understood to relate to any bodily activity that enhances, promotes or maintains physical fitness and overall health, wellbeing and wellness. The physical exercise may be running, jogging, walking, Nordic walking, skating, ice skating, skiing, jumping, rope skipping, spinning, cycling, rowing, weight lifting, football, soccer, basketball, baseball, handball, volleyball, tennis, table tennis, squash, dancing, Zumba®, gymnastics, yoga, aerobics, Pilates, boxing, kickboxing or Tae Bo, for example.

An exercise facility 10 may be, for example, any exercise apparatus or equipment that is usually used for physically exercising. In one aspect, the exercise facility 10 can be any stationary exercise machine such as a stationary exercise bike, treadmill 10', rowing machine, elliptical trainer, cross-trainer, weight machine, skiing machine, climbing machine, stair-climbing machine, or glider machine. In another aspect, the exercise facility 10 may be a mobile exercise device such as a skipping rope, jump rope, suspension training equipment, resistance band, step aerobics platform, punching bag or ball. In yet another aspect, the exercise facility 10 may be a swimming pool 10", climbing wall 10"', dance floor or playing field. Since a large variety of exercise facilities can be combined with the physical exercise apparatus 1 according to the invention, the physical exercise apparatus 1 can be used in almost any conceivable sport setting, making it flexible and easy to adjust to the user's needs.

The measuring device 20 according to the present invention may be any device adapted to determine a variable during a physical exercise. The exact nature of the device 20 may depend on the variable to be determined. In one aspect, the measuring device 20 may be, for example, a sensor suitable for measuring velocity, pressure, distance covered, distance to an object, depth in water or body temperature. The measuring device 20 may comprise electrodes, a clock, a global positioning system (GPS) module, light barriers or a camera.

The variable determined by the measuring device 20 may be representative of any variable related to the physical exercise of the user 2. In an embodiment, it may be representative of a variable depending on the user's body, e.g., a heartrate, sweat factor or temperature of the user 2. A "sweat factor" is herein understood as the amount of sweat produced during the physical exercise. Alternatively, the variable determined may be representative of the physical exercise itself, e.g., the duration or intensity of the physical exercise. The term "intensity" hereby relates to any property of the physical exercise that is suitable to quantify the exertion level of the user 2, e.g., actual or virtual distance covered during the physical exercise, number of calories burnt during the physical exercise, number of repetitions per time unit, weight lifted, or duration during which a movement is held. Herein, "actual or virtual distance covered" relates to a physical exercise involving locomotion. The locomotion may actually take place, e.g., running or swimming from one physical position to another, or may be virtual, e.g., running on a treadmill 10'. Accordingly, the distance covered during the physical exercise may be an actual or a virtual distance. Within the present invention, it is also envisaged to determine one or more variables, for example 2, 3, 4, 5, 6, 7, 8, 9 or 10 variables.

Within the physical exercise apparatus 1 of the present invention, the determined variable is provided to a processing device 30. The processing device 30 of the present invention may be a computing unit and may include a processor on a circuit board, an internal storage unit and connection ports to external storage unit, local area network (LAN) or wireless LAN (WLAN) ports. The processing device 30 is coupled to the measuring device 20 and is adapted to receive one or more inputs and process them via the input unit 310. The one or more variables determined during the physical exercise of the user 2 serve as one or more inputs. The processing device 30 may process the one or more determined variable, for example by calculating the difference between the determined variable and a pre-set value. The pre-set value may be pre-set by the user 2 himself / herself or may be provided to the processing device 30 by other means, for example on the internal storage unit 340 or external storage unit or via any other port. By processing the determined variable, the processing device 30 produces the processed variable, which is transmitted to the output unit 330.

The processing device 30 is furthermore adapted to produce moving graphics with a content depending on the determined or processed variable. "Producing" within the present invention comprises, e.g., "generating", "adjusting", "rendering", "layering" or "zooming". The term "moving graphics" is understood to include animated images, animations, videos, video clips, motion graphics and digital footage.

The content of the moving graphics comprises an object. The term "object" is hereby understood broadly and covers both spatially defined objects and sceneries. In one aspect, the object may be a spatially defined object, e.g., food, clothes, a movable object, person, animal, building or flames. A "person" or "animal" herein refers to actual persons or animals, and fictive ones. In another aspect, the object may be a scenery such as a landscape, scenic view or view of the sky. "Landscape" or "scenic view" hereby refers to actual and fictive landscapes or scenic views, respectively. The object of the moving graphics may be a pursuer 410' such as a lion, bear, tiger, shark, comic villain or alien.

According to the present invention, producing the moving graphics comprises adjusting the size of an object in the content of the moving graphics. The adjustment of the size is performed depending on the determined or processed variable. For example, the size of the object may be increased inversely proportional to the determined or processed variable, or it may be increased proportional to the determined or processed variable.

By adjusting the size of the object in the moving graphics, the user 2 is given the impression that the object changes its position in relation to the user's position. In one embodiment, the impression may be that the distance between the object and the user 2 increases. In another embodiment, the impression may be that the distance between the object and the user 2 decreases. Accordingly, the user 2 of the physical exercise apparatus 1 is given the impression that the physical exercise brings him / her closer to the object or more distant therefrom. In one aspect of the invention, the impression given to the user 2 is that because his / her efforts during physical exercising have decreased, the object has approached and that he / she will distance himself / herself from the object by increasing his / her efforts. In another aspect, the impression given to the user 2 is that, because his / her efforts decreased, the object is farer away and that he / her will approach it by increasing his / her efforts.

It is important to note that the determined variable does not have to be a virtual or actual distance covered by the user for the size of the object to be changed. On the contrary, since the processing device 30 may process any determined variable and thereby provide a processed variable, the processed variable may be used as basis for the adjustment of the size of the object. Accordingly, the physical exercise apparatus 1 of the present invention is adapted to provide the user with an easily understandable representation of his / her achievements during the physical exercise irrespective of the type of physical exercise performed.

The display device 40 is coupled to the processing device 30. The display device 40 may be any type of screen, monitor or display, e.g., a liquid crystal display (LCD), light emitting diode (LED) display, cathode ray tube (CRT) display or plasma display. In one embodiment, it may be an LCD screen. In another embodiment, it may be a projector 40" or a hologram projector. In yet another embodiment, the display device 40 may be a portable device, e.g., a mobile phone, smartphone, tablet PC, laptop or another portable device coupled to a mobile phone, smartphone, tablet PC or laptop. The display device 40 may comprise one or several screens. In a preferred embodiment, the display device 40 comprises one, two or three screens. The display device 40 may be fixed at a certain position and a certain angle in relation to the user 2. In another embodiment, the display device 40 may be movable and / or portable. The display device 40 may comprise rolls, wheels or guide rails attached thereto and, thus, be movable. The display device 40 may also comprise an actuator, e.g., an electric motor, a cable pull or gear wheel which may be disposed on the display device 40 and / or connected thereto. In another embodiment, the display device 40 may be located in front of the user 2 during the physical exercise. In a preferred embodiment the display device 40 is located alongside the user 2, underneath the user 2, above the user 2 or behind the user 2 during the physical exercise.

The display device 40 displays the content of the moving graphics produced by the processing device 30 in response to the determined or processed variable and transmitted via the output unit 330. In an embodiment, the displayed content of the moving graphics may be adapted to induce fear in the user. In another embodiment, the displayed content of the moving graphics is adapted to induce desire in the user. Both fear and desire are powerful emotions that will increase the user's motivation to perform well during the physical exercise involuntarily. In a preferred embodiment, the displayed content of the moving graphics is adapted to give the user 2 the impression to be pursued by a pursuer 410'. In another preferred embodiment, the displayed moving graphics is adapted to give the user 2 the impression to be approaching a reward. It is a well-known principle that "running" away from a pursuer 410' or working towards a reward can set free "hidden" powers within a person; the present invention uses this mechanism to incite the user 2 to give his / her personal best during the physical exercise.

The displayed moving graphics is adapted to increase the interaction of the user 2 with the exercise facility 10. "Increasing the interaction of the user with the exercise facility 10" is herein understood to mean that the user intensifies his / her efforts during the physical exercise. This may result in increased velocity, heartrate, number of calories burnt during the physical exercise, number of repetitions per time unit, weight lifted and / or duration during which a movement is held.

In an embodiment, the invention is adapted to increase an adrenaline level in the user. The adrenaline level may be measured in any suitable sample, e.g., a blood sample or a urine sample. Adrenaline is known to be secreted during exercise, particularly late during exercise when the body accesses its last energy reserves. Thus, by increasing the adrenaline level, the body can be stimulated to mobilize energy reserves.

The processing device 30 may also be adapted to produce sound and coupled to a sound transmitting device 50. The transmitted sound may be a piece of music, song, jingle, sound made by an animal or natural sound. The sound is produced depending on the determined or processed variable, e.g., the produced sound is adapted to the determined variable or processed variable in volume, frequency and / or pitch. Frequency is herein understood as the interval at which a certain sound is produced. In an embodiment, the determined variable is the velocity of the user 2 and the produced sound is louder if the velocity is lower than a pre-set value. In another embodiment, the determined variable is the virtual distance covered by the user 2 during the physical exercise on a treadmill 10' and the produced sound is louder when the distance is larger. The sound transmitting device 50 may be a loudspeaker, headphones, a mobile phone, smartphone or tablet PC. The produced sound provides an additional incentive to the user 2 and creates an immersive environment which enhances motivation of the user 2 during the physical exercise.

The processing device 30 may also be adapted to provide a haptic feedback and coupled to a haptic feedback device 60. "Haptic feedback" is herein understood to comprise any tactile sensation that is provided to the user, e.g., a shaking, vibration, low electric shock, rocking, tickling, etc. The haptic feedback device 60 may be any device capable of transmitting such a haptic feedback, e.g., an electrode, vibration motor, shaking motor, etc. The exact nature of the haptic feedback device 60 depends on the haptic feedback to be transmitted. In an embodiment, the haptic feedback to be transmitted is a low electric shock and the haptic feedback device 60 is an electrode. In another embodiment, the haptic feedback to be transmitted is a vibration and the haptic feedback device 60 is a vibration motor. The haptic feedback is provided dependent on the determined or processed variable, e.g., the haptic feedback is changed in frequency and / or intensity depending on the determined or processed variable. For example, the haptic feedback frequency may increase or decrease depending on the determined or processed variable. The haptic feedback provided to the user 2 additionally enhances the experience of the user 2 during the physical exercise and makes the physical exercise more interesting. Accordingly, the haptic feedback further motivates the user 2.

The physical exercise apparatus 1 according to the invention may also comprise an input device 70 coupled to the processing device 30 and adapted to receive a scenario selected by the user 2 from a plurality of scenarios presented to the user 2. The input device 70 may be any device suitable for presenting the user 2 with a representation of different scenarios, e.g., a touchscreen, screen with buttons, switch, toggle switch, trigger switch, mobile phone, smartphone, tablet computer or laptop computer. The plurality of scenarios may comprise any integer number of scenarios. For example, the plurality of senarios may comprise 2, 3, 4, 5, 6, 7, 8, 9, or 10 scenarios. It is also understood that the selection of a scenario may require a selection for at least one of an object of the moving graphics, a sound and a haptic feedback. By letting the user 2 influence the environment in which he / she performs the physical exercise, the user 2 is further motivated for the physical exercise.

In a second aspect, the present invention relates to a physical exercising method for motivating a user 2 to interact with an exercise facility 10, comprising a) determining, with a measuring device 20, a variable during a physical exercise of the user 2, b) producing, with a processing device 30, moving graphics with a content depending on the determined variable, c) displaying the adapted moving graphics to the user 2, wherein producing the moving graphics involves adjusting the size of an object forming the content of the moving graphics to give the user 2 the impression that the object changes its position relative to the user's position, and the displayed moving graphics increases the interaction of the user 2 with the exercise facility 10.

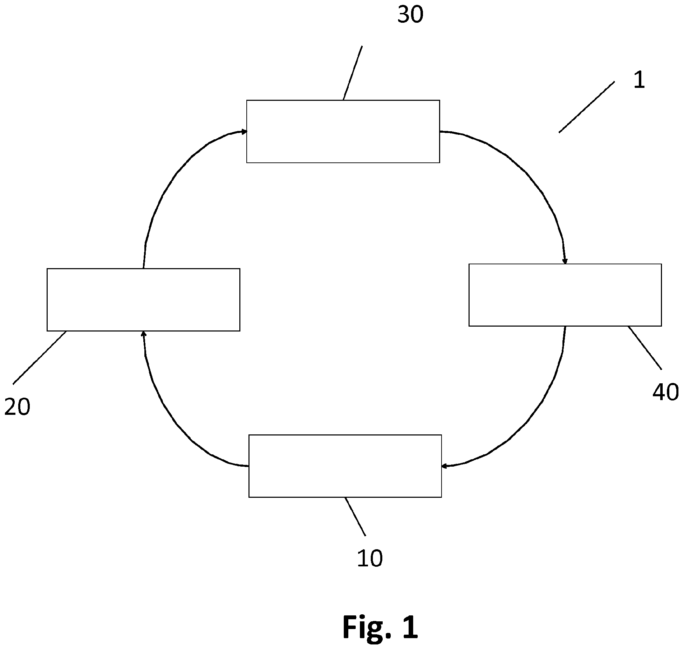

Herein, the variable is determined as described above with reference to Fig. 1. The determined variable is then provided to a processing device 30 which processes the determined variable, which, in turn, is used for producing the moving graphics. The moving graphics comprise an object, the size of which is adjusted according to the determined variable and / or the processed variable. Finally, the object is displayed on a display device 40 so to give the user 40 the impression that the physical exercise brings him / her closer or more distant to the object.

Thus, the method of the present invention provides an effective way for motivating a user 2 during a physical exercise by providing additional incentives that are related to the physical exercise, but not confined to the movements of the exercise themselves. In other words, the user 2 is provided with an adventure that he / she can experience and master while exercising, which increases his / her motivation during the physical exercise.

The invention is illustrated by the following examples, but is defined by the claims.

As described herein, each of the apparatuses and methods according to the present invention may comprise the enumerated features. However, it is also envisaged that apparatuses and methods according to the present invention may consist of the enumerated features.

### Examples

### Example 1: Treadmill with pursuer

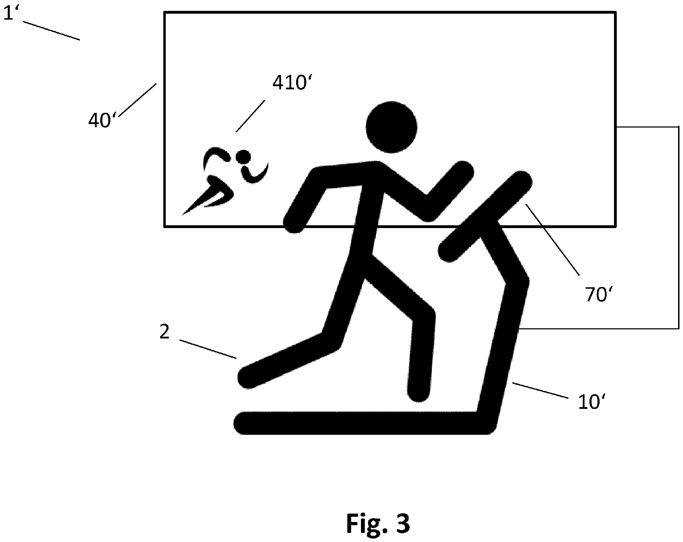

The user 2 wishing to enhance his / her physical exercise experience with a personal motivation uses a treadmill 10' offering a pursuit scenario as shown in Fig. 3. Prior to beginning his / her training session, the user 2 interacts with a touchscreen device as the input device 70' where he / she is presented with a selection of possible pursuers: a tiger, bear, lion, alien or comic villain. After having selected one of the pursuers, the user 2 sets the velocity at which he / she wants to run as well as the intended duration of the exercise. He / She can also choose to perform an interval training with varying velocities.

On a display device next to the treadmill 10', such as a screen 40', the user 2 can see a virtual representation of the selected pursuer 410'. At the beginning of the exercise, the pursuer 410' has a medium size. Additionally, the screen 40' shows the number of (virtual) meters indicating the distance of the pursuer 410' to the user 2 as well as the user's score of virtual points that he / she has collected during this or previous training sessions.

During the exercise, the size of the pursuer 410' changes depending on the velocity of the user 2. Whenever the user 2 lowers his / her velocity, the pursuer 410' on the screen 40' gradually becomes larger, so that the user' has the impression that the pursuer 410' is approaching. When the user 2 increases his / her velocity, the pursuer 410' gradually becomes smaller, so that the user 2 has the impression that he / she is shaking off the pursuer 410'. Additionally, the number of virtual meters indicating the user's lead over the pursuer 410' changes matching the size of the virtual representation of the pursuer 410'.

If the user 2 has chosen an interval training, the size of the pursuer starts changing whenever a new phase of the interval training is reached, so to give the user 2 the impression that the pursuer 410' is changing his velocity and to remind and encourage the user 2 to change his / her velocity accordingly.

Additionally, the user 2 gains virtual points during the training session for every time unit (e.g., 10 seconds) that he / she keeps the pursuer 410' at a certain (virtual) distance. Additionally, he / she gains extra points for every meter that the pursuer 410' is away from him / her when he / she finishes the training session.

### Example 2: Swimming pool with shark

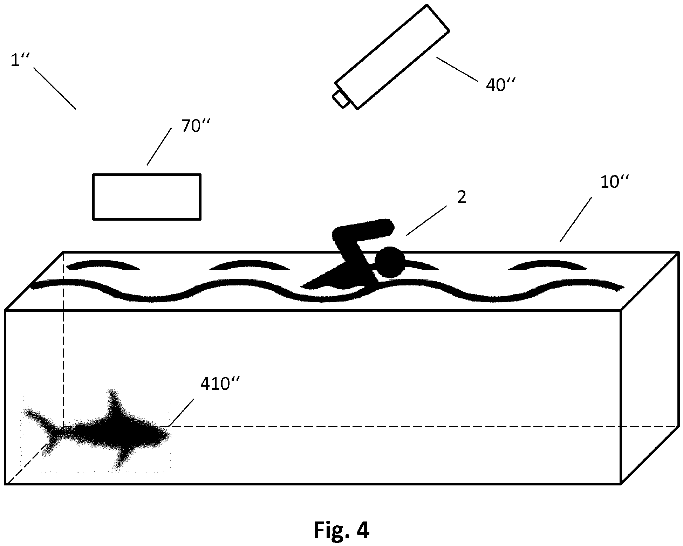

The user 2 wishing to train his swimming skills uses an indoor swimming pool 10" equipped with a projector 40" for projecting a pursuer in shark form 410" as shown in Fig. 4. With the help of a digital input device 70", the user 2 sets the velocity he / she wishes to maintain during the training session. Additionally, he / she can choose whether a tune indicating the distance to the shark ("the shark tune") is played back during the training session.

Once the set-up has been completed, a projection of the pursuer in shark form 410" appears at the bottom of the swimming pool 10".

As soon as the user 2 jumps into the swimming pool 10" and passes a light barrier (not shown), the projection of the pursuer in shark form 410" begins to move and, if applicable, the shark tune is played back. Swimming through the swimming pool 10", the user 2 passes light barriers at regular intervals, allowing to calculate his / her velocity. If the user's velocity is slower that then pre-set velocity, the projection of the shark 410" increases in size; additionally, the frequency or volume of the shark tune increases. If the user's velocity is faster than the pre-set velocity, the projection of the shark 410" decreases in size; additionally, the frequency or volume of the shark tune decreases.

Since the user 2 fears the shark and wants to escape it, he / she increases his / her velocity if he / she notices that the projected shark 410" is approaching, i.e., if the shark projection 410" increases in size. The increased adrenaline production and the thrill of swimming against a shark, thus, increases the user's motivation during the physical exercise.

### Example 3: Climbing wall during a thunderstorm

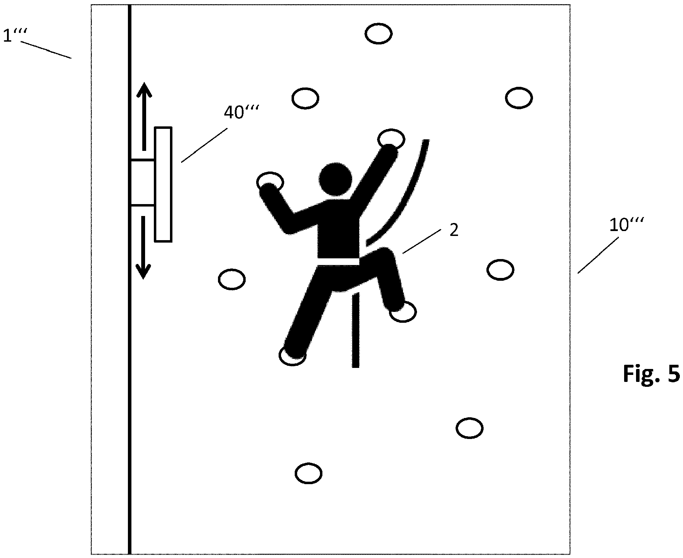

The user 2 wishing to train his / her climbing skills uses a climbing wall 10'" equipped with a movable screen 40'" as shown in Fig. 5. The screen 40'" is movably attached to guide rails and moves up the wall in accordance with the movements of the user 2.

Prior to the training session, the user 2 sets his / her time limit for reaching a specific spot on the climbing wall 10"'. The movable screen 40'" then displays a blue sky with a few small dark clouds in a corner. As time elapses, the clouds on the movable screen 40'" increase in size, giving the user 2 the impression that a thunderstorm is approaching. This is accompanied by the sound of thunder, which increases in volume and frequency with time. The user 2 is motivated to reach the predetermined spot on the climbing wall within the set time since he / she fears the approaching thunderstorms and, thus, has an increased motivation to climb faster.

### List of reference numerals

- 1, 1', 1", 1'": Physical exercise apparatus
- 2: User
- 10: Exercise facility
- 10': Treadmill
- 10": Swimming pool
- 10"': Climbing wall
- 20: Measuring device
- 30: Processing device
- 310: Input unit
- 320: Processing unit
- 330: Output unit
- 340: Storage unit
- 40: Display device
- 40': Screen
- 40": Projector
- 40'": Movable screen
- 410': Pursuer
- 410": Pursuer in the form of a shark
- 50: Sound transmitting device
- 60: Haptic feedback device
- 70, 70', 70": Input device

## Claims

1. A physical exercise apparatus (1; 1'; 1 "; 1"') for motivating a user (2), comprising:
- an exercise facility (10),
- a measuring device (20) adapted to determine a variable during the physical exercise of the user (2),
- a processing device (30) coupled to the measuring device (20) and adapted to produce moving graphics with a content depending on the determined variable, and
- a display device (40) coupled to the processing device (30) and adapted to display the produced moving graphics,
wherein:
- producing the moving graphics comprises adjusting the size of an object in the content of the moving graphics to give the user (2) the impression that the object changes its position relative to the user's position, and
- the displayed moving graphics increases the interaction of the user (2) with the exercise facility (10).

2. The physical exercise apparatus (1; 1'; 1"; 1"') according to claim 1, wherein the object is representative of a pursuer (410'; 410") pursuing the user (2).

3. The physical exercise apparatus (1; 1'; 1 "; 1"') according to claim 1 or 2, wherein the size of the object is inversely proportional to the determined variable.

4. The physical exercise apparatus (1; 1'; 1 "; 1"') according to one of the preceding claims, wherein the size of the object depends on a difference between the determined variable and a pre-set value.

5. The physical exercise apparatus (1; 1'; 1 "; 1"') according to one of the preceding claims, wherein the variable is representative of a heart rate, velocity, sweat factor or temperature of the user (2).

6. The physical exercise apparatus (1; 1'; 1"; 1"') according to one of the preceding claims, wherein the variable is representative of a duration or intensity of the physical exercise.

7. The physical exercise apparatus (1; 1'; 1 "; 1"') according to one of the preceding claims, wherein the displayed moving graphics is adapted to increase an adrenaline level in the user (2).

8. The physical exercise apparatus (1; 1'; 1 "; 1"') according to one of the preceding claims, wherein:
- the processing device (30) is further adapted to produce sound dependent on the determined variable, and
- the physical exercise apparatus (1; 1'; 1 "; 1"') further comprises:
- a sound transmitting device (50) coupled to the processing device (30) and adapted to transmit sound to the user (2).

9. The physical exercise apparatus (1; 1'; 1 "; 1"') according to one of the preceding claims, wherein:
- the processing device (30) is further adapted to produce a haptic feedback dependent on the determined variable, and
- the physical exercise apparatus (1; 1'; 1 "; 1"') further comprises:
- a haptic feedback device (60) coupled to the processing device (30) and adapted to give a haptic feedback to the user (2).

10. The physical exercise apparatus (1; 1'; 1 "; 1"') according to one of the preceding claims, further comprising:
- an input device (70) coupled to the processing device (30) and adapted to receive a scenario selected by the user (2) from a plurality of scenarios presented to the user (2).

11. The physical exercise apparatus (1; 1'; 1 "; 1"') according to one of the preceding claims, wherein the display device (40) is located alongside, above, underneath or behind the user (2).

12. A physical exercising method for motivating a user (2) to interact with an exercise facility (10), comprising:
a) determining, with a measuring device (20), a variable during a physical exercise of the user (2),
b) producing, with a processing device (30), moving graphics with a content depending on the determined variable,
c) displaying the adapted moving graphics to the user (2),
wherein:
- producing the moving graphics involves adjusting the size of an object forming the content of the moving graphics to give the user (2) the impression that the object changes its position relative to the user's position, and
- the displayed moving graphics increases the interaction of the user (2) with the exercise facility (10).

13. The physical exercising method according to claim 12, wherein the object is representative of a pursuer (410'; 410") pursuing the user (2).

14. The physical exercising method according to claim 12 or 13, wherein the size of the object is inversely proportional to the determined variable.

15. The physical exercising method according to one of claims 12 to 14, wherein the size of the object depends on a difference between the determined variable and a pre-set value.

16. The physical exercising method according to one of claims 12 to 15, wherein the determined variable is representative of a heart rate, velocity, sweat factor or temperature of the user (2).

17. The physical exercising method according to one of claims 12 to 15, wherein the determined variable is representative of a duration or intensity of the physical exercise.
